# EUROPÄISCHE PATENTSCHRIFT

(11) **EP 2 345 712 B1**
(45) Veröffentlichungstag und Bekanntmachung des Hinweises auf die Patenterteilung: **27.08.2014**
(21) Anmeldenummer: 11000263.1
(22) Anmeldetag: 14.01.2011
(51) Int. Cl.: C12M 1/107, C12M 1/00, C12M 1/12

(54) **Verfahren zur Fermentation von Presssaft**
Method for fermenting pressed juice
Procédé de fermentation de jus pressé

(30) Priorität: 14.01.2010 DE 102010004736
(43) Veröffentlichungstag der Anmeldung: 20.07.2011
(73) Patentinhaber: GETproject GmbH & Co. KG, 24109 Kiel (DE)
(72) Erfinder: Götz, Johann, 24105 Kiel (DE)
(74) Vertreter: Lobemeier, Martin Landolf

(56) Entgegenhaltungen:
- WO-A1-2007/048537
- WO-A2-2008/142007
- DE-A1-102007 036 729

## Beschreibung

Die Erfindung betrifft ein Verfahren zur Erzeugung von Biogase durch Fermentierung von aus Biomasse hervorgegangenem Presssaft in einem mit einer Vielzahl von Füllkörpern gebildetes Füllkörper-Festbett bestückten Bioreaktor.

Aus der DE 10 2007 036 729 A1 ist bereits ein Verfahren zur Aufbereitung von Biomasse bekannt, bei dem der bei der mechanischen Entwässerung von Biomasse anfallende Presssaft, in dem organischen und anorganischen Pflanzeninhaltsstoffe enthalten sind, dem als Fermenter bezeichneten Bioreaktor einer Biogasanlage zugeführt wird.

Aus dem Dokument DE3726949 A1 ist bereits ein Verfahren zum Betrieb von Festbettreaktoren mit Trägerkörperumlauf bekannt, wobei die behandelnde Flüssigkeit und die Trägerkörper in Gegenstrom durch den Reaktor geführt werden.

Die vergärbaren Stoffe Liegen entweder in gelöster oder im Fall von ungelösten Stoffe in suspendierter Form vor. Dies führt zu einer schnellen Vergärung mit kurzen Verweilzeiten führt, wobei diese ganz wesentlich von der Vergärung der nicht- oder schwerlöslichen Stoffe bestimmt wird. Kurze Verweilzeiten bedeuteten bei den in der Regel kontinuierlich betriebenen Fermentern hohe Durchflussraten mit einem unerwünscht hohen Austrag an Bakterien. Dem wird durch Immobilisierung der Bakterien entgegen gewirkt, indem man ihnen Flächen zum besiedeln anbietet. Dies kann auf verschiedene Arten erfolgen. Beispielsweise sind Bioreaktoren mit einer Schüttung aus Füllkörpern mit großer Oberfläche bekannt. Diese Reaktoren können als Festbett- oder Wirbelbett-Reaktoren ausgeführt werden. Bekannt sind außerdem Reaktoren mit Einbauten aus kreuzweise verschweißten gewellten Folien oder Faservliesmatten mit dazwischen liegenden Strömungskanälen.

Nachteilig an diesen Bioreaktoren ist jedoch, dass die im Presssaft enthaltenen unlöslichen oder schwer löslichen Stoffe, wie z.B. Stärke, die Tendenz aufweisen, Sinkschichten zu bilden, die der Vergärung nur schwer zugänglich sind.

Bei Wirbelbettreaktoren kann die Bildung von Sinkschichten durch eine Verwirbelung des Reaktorinhalts zwar vermieden. Die Verwirbelung führt aber zwangsläufig zu einer Vermischung von frisch zugeführtem Presssaft mit dem Reaktorinhalt. Nachteilig daran ist, dass durch die Vermischung zwangsläufig mit dem Gärrest auch frischer Presssaft ausgetragen wird. Eine hohe Abbaurate lässt sich deswegen bei diesem Verfahren mit Wirbelbetten nur mit großen Fermentern oder durch ein mehrstufiges Verfahren erzielen.

Bei Festbetten mit einer festen Schüttung aus Füllkörpern bilden sich Sinkschichten in den obersten Lagen der Schüttung. Um diese Schichten aufzulösen, ist zumindest von Zeit zu Zeit eine Verwirbelung der Schüttung mit der unerwünschten Folge einer Vermischung von Presssaft und Gärrest notwendig (siehe oben).

Bei Festbetten aus Faserfliesmatten oder Folien mit meist im Bioreaktor vertikal verlaufenden Kanäle bilden sich die Sinkschichten am Boden des Bioreaktors, wobei die Sinkschichten mit teilweise vergorenem Presssaft wieder noch oben gepumpt werden, wodurch es ebenfalls zu einer Vermischung von vergorenem mit frischem Presssaft kommt und die Effizienz des Bioreaktors herabgesetzt ist.

Aufgabe der Erfindung ist es daher, ein Verfahren zur Erzeugung von Biogas durch Fermentierung von aus Biomasse hervorgegangenem Presssaft in einem Bioreaktor zu schaffen, dass die Bildung von für die Fermentation unzugänglichen Sinkschichten vermeidet, ohne dass es zu einer Vermischung von vergorenem und frischem Presssaft kommt.

Die Aufgabe wird erfindungsgemäß durch das Verfahren mit den Merkmalen von Anspruch 1 gelöst. Die Unteransprüche geben vorteilhafte Ausgestaltungen der Erfindung wieder.

Grundgedanke der Erfindung ist es, ein Durchmischen des Bioreaktorinhalts bzw. des Füllkörperbetts vollständig zu vermeiden und stattdessen dem Fülkörperbett an seiner einen Seite eine Teilmenge an Füllkörpern zu entnehmen und der anderen Seite des Füllkörperbetts zuzuführen. Dabei ist diejenige Seite, der die Füllkörper zugeführt werden - unabhängig davon, ob der Bioreaktor vom Presssaft von oben nach unten oder von unten nach oben durchströmt wird - auf der der Fließrichtung des Presssafts entgegen gesetzten Seite des Bioreaktors angeordnet. Bei nicht schwimmfähigen Füllkörpern werden die entnommenen Teilmengen also auf das Fütlkörper-Festbett aufgebracht und bei schwimmfähigen Füllkörpern werden die Teilmengen unterhalb des im Bioreaktor aufschwimmenden Füllkörper-Festbetts in den Reaktor eingebracht.

Die Erfindung wird anhand von in den Zeichnungen dargestellten Ausführungsbeispielen näher erläutert. Es zeigen:
- Fig. 1: ein erstes Ausführungsbeispiel eines zur Verwendung des erfindungsgemäßen Verfahrens eingerichteten Bioreaktors;
- Fig. 2: ein zweites Ausführungsbeispiel eines zur Verwendung des erfindungsgemäßen Verfahrens eingerichteten zweiten Bioreaktors; und
- Fig. 3: einen schematisch dargestellten Verfahrensahlauf für den in Fig. 1 dargestellten Bioreaktor.

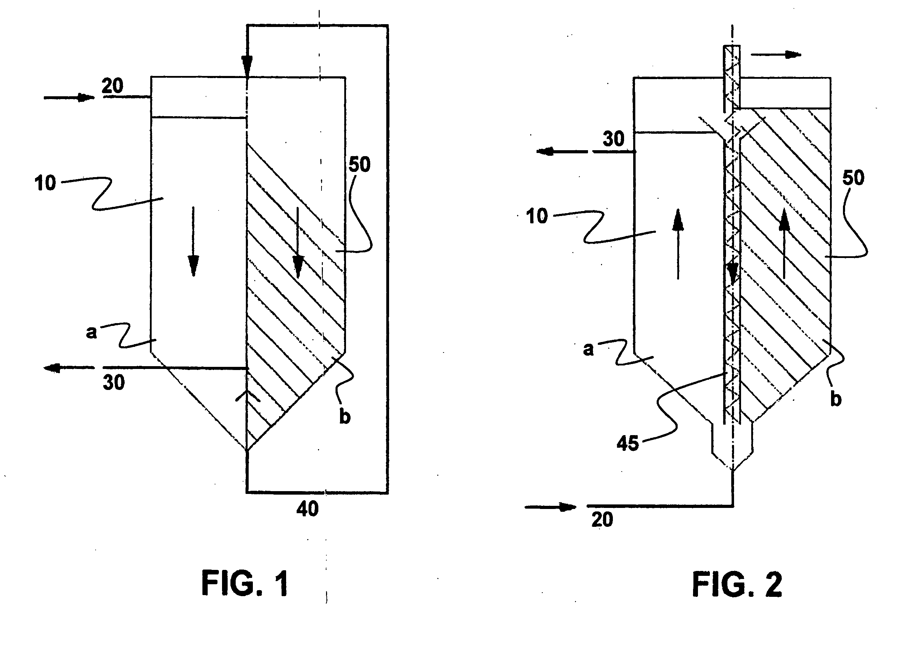

Fig. 1 zeigt einen für zur Durchführung des Verfahrens geeigneten Bioreaktor 10. Der Bioreaktor 10 weist in seinem oberen Bereich einen Zulauf 20 zum Einleiten von Presssaft in den Bioreaktor 10 und in seinem unteren Bereich einen Ablauf 30 zur Entnahme der Gärreste auf. Der Innenraum des Bioreaktors 10 ist mit einer Schüttung von nicht schwimmfähigen Füllkörpern 50 bis zu einem vorbestimmten Füllstand gefüllt, die zur effizienten Ansiedlung mit einer möglichst hohen Menge Mikroorganismen geeignet sind. Die Füllkörper sind darüber hinaus so geformt, dass sich auf ihnen Sinkstoffe absetzen können.

Darüber hinaus weist der Bioreaktor 10 in seinem bevorzugt konisch ausgebildeten Bodenbereich eine mit dem Inhalt des Bioreaktors 10 verbundene Fördereinrichtung 40 auf, über die die im unteren Bereich des Bioreaktors 10 befindlichen Füllkörper abgezogen und dem Bioreaktor 10 auf seiner Oberseite wieder zugeführt werden können. Wahlweise kann der Bioreaktor auch mit einer zentral angeordneten Fördereinrichtung für die Umwälzung der Füllkörper ausgerüstet werde.

Wird nun im oberen Bereich des Bioreaktors 10 Presssaft zugeführt, bevorzugt oberhalb der Schüttung aus Füllkörpern, wird gleichzeitig eine definierte Menge an Füllkörpern über die Fördereinrichtung 40 aus dem unteren Bereich des Bioreaktors abgezogen und oben auf die Schüttung der Füllkörper aufgebracht. Die mit dem Presssaft eingebrachten Sinkstoffe lagern sich in den obersten Schichten der mit Bakterien besetzten Füllkörper ab und bilden dabei gleichmäßig dünne Schichten, wodurch die nicht- bzw. schwerlöslichen Stoffe den Bakterien leicht zugänglich sind, was zu einer effektiven Vergärung führt. Presssaft und Füllkörper wandern so beide von oben nach unten durch den Bioreaktor. Die Menge an Füllkörpern und die Geschwindigkeit des Presssaft und der Füllkörpern im Bioreaktor 10 sind jeweils so bemessen, dass die Gärung beim Erreichen des unteren Bereiches des Bioreaktors 10 abgeschlossen ist. Nur vollständig vergorener Presssaft wird aus dem Bioreaktor abgezogen.

In Fig. 1 ist gezeigt, wie der Presssaft und die nicht schwimmfähigen Füllkörper mit unterschiedlicher Geschwindigkeit von oben nach unten durch den Bioreaktor geführt werden, wobei der mit a bezeichnete Presssaft auf der linken Seite und die mit b bezeichnet Schüttung von Füllkörpern auf der rechten Seite dargestellt.

In Fig. 2 ist ein Bioreaktor mit schwimmfähigen Füllkörpern dargestellt, bei dem sich Presssaft von unten oben bewegen. Der Presssaft wird dem Bioreaktor 10 von unten zugeführt. Beispielhaft ist dargestellt, wie durch eine Förderschnecke 45 die Füllkörper von oben nach unten befördert werden. Durch eine Drehrichtungsumkehr der Förderschnecke 45 können Füllkörper dem Bioreaktor entnommen werden. Die Zuführung des Presssaftes ist so ausgebildet, dass auf Grund der aufwärts gerichteten Strömung und einer ausreichenden Strömungsgeschwindigkeit des Presssaftes sich keine Sinkschichten am Boden des Bioreaktors absetzen können.

Bevor die Füllkörper erneut in den Bioreaktor eingebracht werden, können sie gereinigt oder so konditioniert werden dass das Wachstum von spezifischen Mikroorganismengesellschaft auf den Füllkörpern begünstigt wird. Außerdem können mit Mikroorganismen besiedelte Füllkörper ausgetragen und wieder in den Fermenter eingebracht werden, um so die Gasproduktion kurzfristig an einen geänderten Bedarf anzupassen. Auf diese Weise kann die Fermentation des Presssafts stets an einem Optimum betrieben werden.

Dabei kann das Einleiten von Presssaft, das Abziehen von Füllkörpern und das Zuführen von Füllkörpern chargenweise oder kontinuierlich erfolgen, wobei sich die Füllkörper mit im zeitlichen Mittel konstanter Sinkgeschwindigkeit durch den Bioreaktor bewegen sollen.

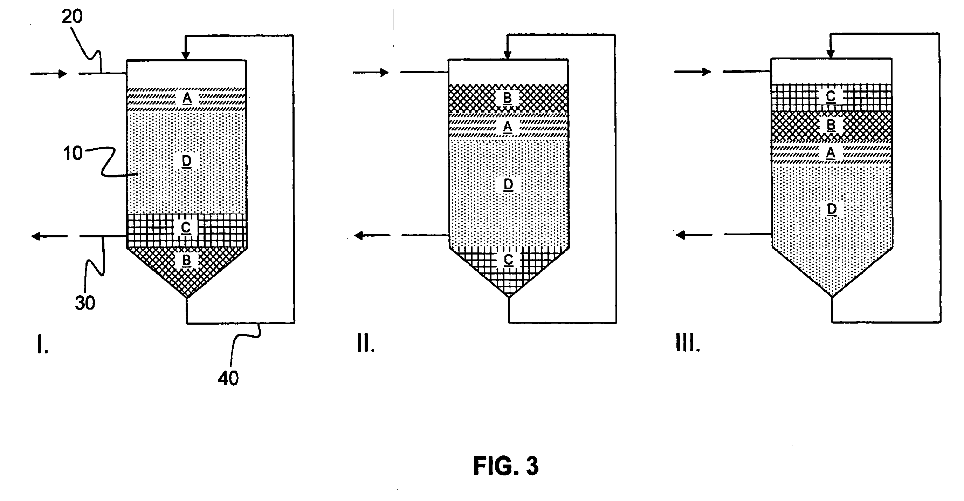

Fig. 3 zeigt den erfindungsgemäßen Verfahrensablauf in einer rein schematischen Darstellung in drei aufeinander folgenden Phasen I, II, III bei chargenweiser Entnahme einer vorbestimmten Teilmenge an Füllkörpern aus dem Bioreaktor 10. Fig. 3 zeigt den aus Fig. 1 bekannten Bioreaktor 10, der eine im Folgenden als Füllkörperbett bezeichnete Schüttung von Füllkörpern aufweist (wobei auf die Darstellung eines Schüttkegels verzichtet ist).

Das Füllkörperbett ist zum Zweck der Erläuterung der Erfindung willkürlich in vier Teilmengen A, B, C, D eingeteilt, die horizontale Schichten darstellen. Abweichend von der Darstellung können diese Teilmenge auch ein gleich großes Volumen oder eine gleich mächtige Schichtdicke aufweisen.

An die im Reaktor 10 zuoberst angeordnete Teilmenge A schließen sich in Fließrichtung des Presssafts (von oben nach unten) eine Teilmenge D, Teilmenge C und Teilmenge B an. Die Teilmengen A, B, C, D bilden das Füllkörperbett, wobei bevorzugt der Zulauf 20 oberhalb der Oberfläche des Füllkörperbetts und der Ablauf 30 bevorzugt oberhalb der Entnahme der Füllkörper am Boden des Bioreaktors 10 angeordnet ist.

Wird die Teilmenge B dem Bioreaktor 10 am Boden des Bioreaktors 10 entnommen (siehe Fig. 3 II), sackt das verbleibende Füllkörperbett, nämlich die Teilmengen A, D und C, ab. Bevorzugt gleichzeitig wird die entnommene Teilmenge B auf die Oberfläche des Füllkörperbetts aufgebracht, sodass das Füllkörperbett insgesamt sein Volumen beibehält.

Darauf kann in einem weiteren Schritt dem Bioreaktor 10 die Teilmenge C entnommen werden und auf die nun durch die Teilmenge B gebildete Oberfläche des Füllkörperbetts aufgebracht werden.

Die Entnahme und das Aufbringen der Teilmengen soll so erfolgen, dass es zu keiner Durchmischung von frisch zu geführtem Presssaft und vergorenem Presssaft kommt. Dabei kann die Geschwindigkeit, mit der die Füllkörper durch den Bioreaktor 10 wandern mit der Fließgeschwindigkeit des Presssafts im Bioreaktor 10 identisch sein oder von dieser abweichen, z.B. geringer sein. Es ist lediglich darauf zu achten, dass die Wandergeschwindigkeit der Füllkörper im Bioreaktor 10 so bemessen ist, dass die sich auf den Füllkörpern in dünnen Schichten absetzenden Sinkstoffe beim Durchwandern der Füllkörper durch den Bioreaktor 10 im Wesentlichen vollständig abgebaut werden.

Bei dem erfindungsgemäße Verfahren wird ein Bioreaktor gleichmäßig in gleicher Richtung von Presssaft und Füllkörpern durchströmt, wobei sich die in der Flüssigkeit nach unten sinkende, nicht gelösten Stoffe, insbesondere Stärke, gleichmäßig in dünnen Schichten auf den mit Mikroorganismen besetzten Flächen der Füllkörpern ablagern. Die dünnen Schichten dieser Stoffe sind auf diese Weise für die Mikroorganismen einfach zugänglich und können so von diesen leicht abgebaut werden. Die Menge der Füllkörper und deren Geschwindigkeit im Bioreaktor werden so gewählt, dass die vergärbaren Stoffe abgebaut sind, wenn die sie tragenden Füllkörper den Bioreaktors durchwandert haben. Eine Vermischung von ausgegorenem und frischem Presssaft wird vermieden und damit die Abbaurate erhöht.

## Patentansprüche

1. Verfahren zur Erzeugung von Biogas durch Fermentierung von aus Biomasse hervorgegangenem Presssaft in einem Bioreaktor (10), wobei der Bioreaktor (10) ein aus einer Vielzahl von Füllkörpern gebildetes Füllkörper-Festbett aufweist, mit den Schritten:
a) Einleiten von Presssaft in den Bioreaktor (10), und
b) Abziehen von fermentiertem Presssaft aus dem Bioreaktor (10),
**gekennzeichnet durch** die weiteren Schritte:
c) Entnehmen einer Teilmenge (A, B, C, D) der Füllkörper aus dem Bereich des Bioreaktors (10), in dem der fermentierte Presssaft aus dem Bioreaktor (10) abgezogen wird,
d) Zuführen der Teilmenge (A, B, C, D) der Füllkörper in den Bereich des Bioreaktors, in dem der Presssaft in den Bioreaktor (10) eingeleitet wird, und
e) Wiederholen der Schritte c) und d),
wobei die zugeführte Teilmenge (A, B, C, D) der Füllkörper auf diejenige Oberfläche des Füllkörper-Festbetts aufgebracht wird, die der Fließrichtung des Presssafts im Bioreaktor (10) entgegen gesetzt ist.

2. Verfahren nach Anspruch 1, **dadurch gekennzeichnet, dass**
- die Füllkörper nicht schwimmfähig sind, und
- der Presssaft im oberen Bereich des Bioreaktors (10) in den Bioreaktor (10) eingeleitet und im unteren Bereich des Bioreaktors (10) aus dem Bioreaktor (10) abgezogen wird,
wobei die entnommene Teilmenge (A, B, C, D) der Füllkörper im oberen Bereich des Bioreaktors (10) auf das Füllkörper-Festbett aufgebracht wird.

3. Verfahren nach Anspruch 1, **dadurch gekennzeichnet, dass**
- die Füllkörper schwimmfähig sind, und
- der Presssaft im unteren Bereich des Bioreaktors (10) in den Bioreaktor (10) eingeleitet und im oberen Bereich des Bioreaktors (10) aus dem Bioreaktor (10) abgezogen wird,
wobei die entnommene Teilmenge (A, B, C, D) der Füllkörper in den unteren Bereich des Bioreaktors (10) unterhalb des Füllkörper-Festbetts zugeführt wird.

4. Verfahren nach einem der Ansprüche 1 bis 3, **dadurch gekennzeichnet, dass** das Einleiten von Presssaft, das Abziehen von fermentiertem Presssaft, das Abziehen von Füllkörpern und das Zuführen von Füllkörpern kontinuierlich erfolgt.

5. Verfahren nach einem der vorhergehenden Ansprüche, **dadurch gekennzeichnet, dass** die pro Zeiteinheit entnommene Teilmenge (A, B, C, D) an Füllkörpern und/oder die pro Zeiteinheit zugeführte Teilmenge (A, B, C, D) an Füllkörpern in einer durchschnittlichen Wandergeschwindigkeit der Füllkörper durch den Bioreaktor (10) resultiert, die geringer als die Fließgeschwindigkeit des Pressafts im Bioreaktor (10) ist.

6. Verfahren nach einem der vorhergehenden Ansprüche, **dadurch gekennzeichnet, dass** die entnommene Teilmenge (A, B, C, D) der Füllkörper vor dem Zuführen mit einem Nähr- oder Konditionierungsmedium für Mikroorganismen in Kontakt gebracht und/oder von störendem Bewuchs befreit wird.

7. Verfahren nach einem der vorhergehenden Ansprüche, **dadurch gekennzeichnet, dass** dem Bioreaktor (10) mit Mikroorganismen besiedelte Füllkörper entnommen und zugeführt werden.

## Claims

1. A method for producing biogas by fermentation of pressed juice, produced from biomass, in a bioreactor (10), the bioreactor (10) exhibiting a filling material-packed bed formed by a multiplicity of filling material, having the steps:
a) introducing pressed juice into the bioreactor (10), and
b) withdrawing fermented pressed juice from the bioreactor (10),
**characterized by** the further steps:
c) removing a partial amount (A, B, C, D) of the filling material from the area of the bioreactor (10), in which the fermented pressed juice is withdrawn from the bioreactor (10),
d) supplying the partial amount (A, B, C, D) of the filling material into the area of the bioreactor in which the pressed juice is introduced into the bioreactor (10), and
e) repeating the steps c) and d),
the supplied partial amount (A, B, C, D) of the filling material being applied to that surface of the filling material-packed bed that is counter to the flow direction of the pressed juice in the bioreactor (10).

2. The method according to Claim 1, **characterized in that**
- the filling material cannot float and
- the pressed juice is introduced in the upper area of the bioreactor (10) into the bioreactor (10) and is withdrawn in the lower area of the bioreactor (10) from the bioreactor (10),
the partial amount (A, B, C, D) of the filling material removed is applied in the upper area of the bioreactor (10) to the filling material-packed bed.

3. The method according to Claim 1, **characterized in that**
- the filling material can float and
- the pressed juice is introduced in the lower area of the bioreactor (10) into the bioreactor (10) and is withdrawn in the upper area of the bioreactor (10) from the bioreactor (10),
the partial amount (A, B, C, D) of the filling material removed is fed into the lower area of the bioreactor (10) below the filling material-packed bed.

4. The method according to one of Claims 1 to 3, **characterized in that** introducing pressed juice, withdrawing fermented pressed juice, withdrawing filling material and feeding filling material takes place continuously.

5. The method according to one of the preceding claims, **characterized in that** the partial amount (A, B, C, D) of filling material removed per unit time and/or the partial amount (A, B, C, D) of the filling material fed per unit time results in an average migration velocity of the filling material through the bioreactor (10) that is lower than the flow velocity of the pressed juice in the bioreactor (10).

6. The method according to one of the preceding claims, **characterized in that**, prior to feeding, the partial amount (A, B, C, D) of the filling material removed is contacted by a culture and conditioning medium for microorganisms and/or is freed from interfering vegetation.

7. The method according to one of the preceding claims, **characterized in that** filling material populated with microorganisms is removed from and fed to the bioreactor (10).

## Revendications

1. Procédé de fabrication de biogaz par fermentation de jus de pressage obtenu dans un bioréacteur (10) à partir de biomasse, à savoir que le bioréacteur (10) présente un lit fixe constitué d'une multitude de d'éléments de remplissage. La fabrication implique les étapes suivantes :
a) Introduction du jus de pressage dans le bioréacteur (10), et
b) retrait hors du bioréacteur (10) du jus de pressage fermenté,
**caractérisé par** les étapes suivantes :
e) Retrait d'une quantité partielle (A, B, C, D) des éléments de remplissage hors du bioréacteur (10), par le biais du retrait hors du bioréacteur (10) du jus de pressage fermenté.
d) Ajout de la quantité partielle (A, B, C, D) des éléments de remplissage au niveau du bioréacteur en introduisant le jus de pressage dans le bioréacteur (10), et
e) réitération des étapes c) et d),
à savoir que la quantité partielle introduite (A, B, C, D) des éléments de remplissage est appliquée sur la surface du lit fixe à éléments de remplissage qui est placé, dans le bioréauteur (10), dans la direction d'écoulement du jus de pressage opposée.

2. Procédé selon la revendication 1, **caractérisé en ce que**
- les éléments de remplissage ne sont pas flottants, et
- le jus de pressage situé dans la partie supérieure du bioréacteur (10) est introduit dans le bioréacteur (10), et est retiré, dans la partie inférieure du bioréacteur (10), du bioréacteur (10).
à savoir que la quantité partielle prélevée (A, B, C, D) d'éléments de remplissage situés dans la partie supérieure du bioréacteur (10) est appliquée sur le lit solide d'éléments de remplissage.

3. Procédé selon la revendication 1, **caractérisé en ce que**
- les éléments de remplissage sont flottants, et
- le jus de pressage situé dans la partie inféreure du bioréacteur (10) est introduit dans le bioréacteur (10), et est retiré, dans la partie supérieure du bioréacteur (10), du bioréacteur (10).
à savoir que la quantité partielle prélevée (A, B, C, D) d'éléments de remplissage situés dans la partie inférieure du bioréacteur (10) est conduite sous le lit solide d'éléments de remplissage.

4. Procédé selon une des revendications 1 à 3 **caractérisé en ce que** l'introduction de jus de pressage, le trait du jus de pressage fermenté, le retrait des éléments de remplissage et l'apport d'éléments de remplissage se fait en continu.

5. Procédé selon une des revendications précédentes, **caractérisé en ce que** la quantité partielle d'éléments de remplissage retirées par unité de temps (A, B, C, D) et/ou la quantité partielle d'éléments de remplissage amenée par unité de temps (A, B, C, D) a pour conséquence une vitesse de migration moyenne des éléments de remplissage par le bioréacteur (10) inférieure à la vitesse d'écoulement du jus de pressage dans le bioréacteur (10).

6. Procédé selon une des revendications ci-avant, **caractérisé en ce que** la quantité partielle d'éléments de remplissage prélevée (A, B, C, D) est mise en contact, avant l'introduction, avec un agent nourrissant ou de conditionnement pour micro-organismes et/ou libéré des colonisations parasites.

7. Procédé selon une des revendications ci-avant, **caractérisé en ce que** des éléments de remplissages colonisés par des micro-organismes sont retirés et amenés au bioréacteur (10).
